## Europäisches Patentamt

## European Patent Office

## Office européen des brevets

(19)

(11) Veröffentlichungsnummer: **0 052 285**
**B1**

(12) **EUROPÄISCHE PATENTSCHRIFT**

(45) Veröffentlichungstag der Patentschrift:
28.09.83

(51) Int. Cl.³: **C 07 C 53/21,** C 07 C 51/305 //
B01F3/08, B01F17/00

(21) Anmeldenummer: 81109322.8

(22) Anmeldetag: 30.10.81

(54) Verfahren zur Reindarstellung gesättigter Perfluoralkancarbonsäuren(1) aus 1-Iodperfluoralkanen.

(30) Priorität: 15.11.80 DE 3043249

(43) Veröffentlichungstag der Anmeldung:
26.05.82 Patentblatt 82/21

(45) Bekanntmachung des Hinweises auf die Patenterteilung:
28.09.83 Patentblatt 83/39

(84) Benannte Vertragsstaaten:
BE CH DE FR GB IT LI NL

(56) Entgegenhaltungen:
DE-A-2 207 177
DE-B-1 211 619
DE-B-1 518 699

(73) Patentinhaber: HOECHST AKTIENGESELLSCHAFT,
Postfach 80 03 20, D-6230 Frankfurt am Main 80 (DE)

(72) Erfinder: von Werner, Konrad, Dr., Talhausen 11 1/3,
D-8261 Burgkirchen/Alz (DE)
Erfinder: Gisser, Alfons, Max-Planck-Platz 8,
D-8261 Burgkirchen/Alz (DE)

# 0 052 285

## Verfahren zur Reindarstellung gesättigter Perfluoralkancarbonsäuren(1) aus 1-Iodperfluoralkanen

Die Erfindung betrifft ein Verfahren zur Herstellung von reinen, insbesondere geradkettigen Perfluorcarbonsäuren aus 1-Iodperfluoralkanen in hoher Ausbeute. Gegenstand der Erfindung ist weiterhin eine wirksame Methode zur Wiedergewinnung des Iods.

Perfluorcarbonsäuren sind nicht-oxidierende, starke Säuren, welche sich durch eine hervorragende thermische und chemische Stabilität auszeichnen und, bedingt durch den Perfluoralkylrest, gute oberflächenaktive Eigenschaften besitzen.

Die perfluorierten Carbonsäuren eignen sich als Verdampfungsunterdrücker bei flüchtigen, brennbaren organischen Flüssigkeiten. In Form ihrer Ammonium- oder Alkalisalze werden sie als Emulgatoren, beispielsweise bei der Polymerisation fluorierter Alkene, zum Beispiel von Tetrafluorethylen, verwendet. Die fluorierten Carbonsäuren stellen darüber hinaus wertvolle Zwischenprodukte zur Herstellung anderer Fluortenside dar, insbesondere von öl- und wasserabweisenden Mitteln.

Es ist bekannt, Perfluorcarbonsäuren durch Hydrolyse entsprechender Säurehalogenide herzustellen, welche aus fluorfreien Vorprodukten durch elektrochemische Fluorierung zugänglich sind (Chemiker-Zeitung, Heft Nr. 1 des Jahres 1976, Seite 6). Dieses Verfahren ist jedoch wegen der Verwendung von flüssigem Fluorwasserstoff nicht ungefährlich und liefert, speziell bei langkettigen Carbonsäuren, niedrige Ausbeuten.

Ein anderes bekanntes Verfahren zur Herstellung von Perfluorcarbonsäuren besteht darin, daß Perfluoralkylmagnesiumhalogenide mit Kohlendioxid (Journal of the Chemical Society, Jahr 1952, Seite 3423) oder Dialkylcarbonaten (deutsche Patentschrift 1 926 942) umgesetzt werden. Nachteilig ist dabei jedoch die Thermolabilität der Fluor-Alkyl-Grignard-Verbindungen, die technische Schwierigkeiten bereitet.

Es ist ferner bekannt, daß Perfluorcarbonsäuren durch Reaktion von Perfluorhalogenalkanen mit gasförmigem Kohlendioxid und einem Zink-Metallpaar der Formel $ZnM^2$, worin $M^2$ ein elektropositiveres Metall im Vergleich zu Zink ist, unter Verwendung von Dialkylsulfoxiden als Lösemittel hergestellt werden können (deutsche Offenlegungsschriften 2 708 751 und 2 756 169). Ein Nachteil dieses Herstellungsverfahrens besteht in der geringen Beständigkeit der Dialkylsulfoxide gegenüber Reduktionsmitteln. Es werden daher Perfluorcarbonsäuren erhalten, denen selbst nach destillativer Reinigung der üble Geruch von Dialkylsulfid anhaftet. Ein ähnliches Verfahren benutzt substituierte Carbonsäureamide als Lösungsmittel, wobei zunächst Addukte der Perfluorcarbonsäuren mit den verwendeten Carbonsäureamiden entstehen, aus denen die Säure in einem separaten Schritt freigesetzt werden muß (deutsche Offenlegungsschrift 2 848 197).

Ebenso ist es bekannt, daß Perfluorcarbonsäuren durch Oxidation von Perfluorolefinen (Journal of American Chemical Society, Band 75, Seiten 2698 bis 2702), beziehungsweise von Perfluoralkanen mit $CCl_3$-, $CFCl_2$- oder $CFClBr$-Endgruppen (US-Patentschriften 2 806 865 und 2 806 866) hergestellt werden können. Diese Verfahren sind jedoch teuer und im technischen Maßstab schwer durchführbar.

Weiterhin ist aus DE-PS 1 211 619 bekannt, daß man bei der Umsetzung von 1-Iodperfluoralkanen mit rauchender Schwefelsäure Perfluorcarbonsäuren neben den entsprechenden Säurefluoriden erhält. Die in der genannten Patentschrift angegebenen Beispiele beschreiben nur Reaktionen von verzweigtkettigen Ausgangsverbindungen. Nach der Reaktion mit dem 1-Iodperfluoralkan soll die abgetrennte rauchende Schwefelsäure für eine erneute Reaktion mit 1-Iodperfluoralkan wieder einsetzbar sein, nachdem Schwefeltrioxid zugegeben und filtriert wurde.

Allgemein besteht ein Bedarf für Verfahren, mit deren Hilfe die aufgrund ihrer weiter oben beschriebenen günstigen Eigenschaften geschätzten Perfluorcarbonsäuren mit 6 bis 14 C-Atomen hergestellt werden können. Als Ausgangsprodukte hierfür geeignet sind insbesondere geradkettige 1-Iodperfluoralkane der Formel $CF_3(CF_2)_xI$, worin x eine ganze Zahl von 5 bis 13 bedeutet, da solche Verbindungen leicht durch Telomerisation von Iodtrifluormethan oder Iodpentafluorethan mit Tetrafluorethylen zugänglich sind.

Aufgabe der vorliegenden Erfindung ist es, ein Verfahren zur Verfügung zu stellen, das es gestattet, die genannten Perfluorcarbonsäuren mit guter Ausbeute und Reinheit unter weitgehender Wiedergewinnung des wertvollen elementaren Iods wirtschaftlich günstig zu gewinnen.

Diese Aufgabe wird gelöst durch ein Verfahren zur Reindarstellung gesättigter Perfluoralkancarbonsäuren(1) mit 6 bis 14 C-Atomen aus den entsprechenden 1-Iodperfluoralkanen unter weitgehender Rückgewinnung von Iod durch Umsetzung der 1-Iodperfluoralkane mit rauchender Schwefelsäure, die 15 bis 45 Gew.-%, bezogen auf die Lösung, Schwefeltrioxid gelöst enthält, bei Temperaturen von 100 bis 180°C, gegebenenfalls unter Druck, wobei das Molverhältnis von Schwefeltrioxid zum 1-Iodperfluoralkan 2 : 1 bis 15 : 1 beträgt und nach beendeter Reaktion die entstandenen zwei flüssigen Phasen voneinander getrennt werden, das dadurch gekennzeichnet ist, daß man die leichtere, flüssige Phase mit 0,3 bis 6% ihres Gewichtes Wasser oder einer entsprechenden Menge einer wasserhaltigen Flüssigkeit, gegebenenfalls bei erhöhter Temperatur, vermischt und anschließend diese Mischung bei 0,1 bis 100 kPa Druck destilliert, ferner die nach der Reaktion mit rauchender Schwefelsäure abgetrennte, schwerere, flüssige Phase mit 0,7 bis 1,5 Mol Wasser oder

2

einer entsprechenden Menge einer wasserhaltigen Schwefelsäure je Mol eingesetztes Schwefeltrioxid, gegebenenfalls unter zusätzlicher Erwärmung, vermischt, danach auf 0 bis +30°C abkühlt, das ausgeschiedene feste Iod abfiltriert, mit wasserhaltiger Schwefelsäure sowie mit Wasser wäscht und gegebenenfalls nach bekannten Methoden trocknet und reinigt.

Die rauchende Schwefelsäure soll zwischen 15 und 45 Gew.-%, vorteilhaft zwischen 30 und 40 Gew.-%, Schwefeltrioxid enthalten. Bei einem Schwefeltrioxid-Gehalt unter 15 Gew.-% ist die Reaktion sehr langsam, bei einem Gehalt über 45 Gew.-% treten störende Nebenreaktionen auf.

Man führt die Umsetzung bei mindestens 100°C durch, damit sie mit ausreichender Geschwindigkeit abläuft. Andererseits sollten Temperaturen oberhalb 180°C vermieden werden, um Nebenreaktionen, wie zum Beispiel Crackprozesse an der Perfluoralkylkette, zu verhindern. Bevorzugt wird bei 130 bis 170°C gearbeitet, der optimale Bereich liegt zwischen 140 und 160°C.

Das Molverhältnis von eingesetztem Schwefeltrioxid zu 1-Iodperfluoralkan soll 2 : 1 bis 15 : 1 betragen. Bei einem Verhältnis kleiner 2 läßt sich auch mit sehr langen Reaktionszeiten kein ausreichender Umsatz erzielen, während ein Verhältnis größer 15 nicht nur mit einer Verwendung von unnötig viel rauchender Schwefelsäure verbunden ist, sondern auch durch zunehmende Seitenreaktionen zu einer Ausbeuteverschlechterung führt. Insbesondere für die Umsetzung von Schwefeltrioxid mit 1-Iodperfluoralkanen, die 6 bis 14 C-Atome enthalten, sind Molverhältnisse von 3 : 1 bis 6 : 1 vorteilhaft.

Die Reaktion des 1-Iodperfluoralkans mit rauchender Schwefelsäure wird am besten in einem geschlossenen System vorgenommen, um ein Entweichen von Schwefeltrioxid oder Iodperfluoralkan zu verhindern. Als Reaktionsgefäß eignet sich beispielsweise ein Kessel oder Autoklav aus Stahl. Die Stahloberfläche wird unter dem Einfluß des Schwefeltrioxids sowohl in der Flüssig- als auch in der Gasphase passiviert. Es bildet sich eine Schutzschicht aus, die eine Korrosion verhindert.

Es ist günstig, die Reaktionspartner während der Umsetzung intensiv zu durchmischen, was nach üblichen Methoden, beispielsweise durch Rühren oder Schütteln erreicht werden kann.

Je nach gewählter Temperatur und Kettenlänge des eingesetzten 1-Iodperfluoralkans ist die Reaktion im allgemeinen in etwa 1 bis etwa 12 Stunden abgeschlossen. Bei Temperaturen im Bereich von 130 bis 170°C ist bei mittlerer Kettenlänge des 1-Iodperfluoralkans die Reaktion in 4 bis 8 Stunden beendet. Bei längerkettigen 1-Iodperfluoralkanen empfiehtl es sich, die Reaktionsdauer um 1 bis 3 Stunden zu verlängern.

Das Reaktionsgemisch trennt sich beim Abkühlen auf etwa 20°C meist glatt in zwei Phasen, die wegen ihrer unterschiedlichen Farbe leicht separiert werden können. Die hellgefärbte leichtere Phase besteht im wesentlichen aus dem Säurefluorid $CF_3(CF_2)_{x-1}COF$ und der Carbonsäure $CF_3(CF_2)_{x-1}COOH$ (x hat die obengeannte Bedeutung). Die dunkelbraune schwerere Phase besteht hauptsächlich aus Schwefelsäure, Schwefeltrioxid, Fluorsulfonsäure, Iod, Iodwasserstoff und Schwefeldioxid.

Bei der Herstellung von relativ kurzkettigen Carbonsäuren, insbesondere von Perfluorhexansäure, lösen sich die Fluorprodukte merklich in der schwereren Phase. Sie können aus dieser mit Hilfe von Lösungsmitteln extrahiert werden, welche nicht mit rauchender Schwefelsäure mischbar sind, nicht mit dieser reagieren und einen Siedepunkt bei 98 kPa von 40 bis etwa 150°C aufweisen, beispielsweise mit hochfluorierten oder perfluorierten, gesättigten aliphatischen Kohlenwasserstoffen, Ethern oder Chlorfluorkohlenwasserstoffen, wie Perfluor-n-hexan; Perfluorcyclohexan; Perfluor-n-heptan; Perfluorhexylmethyl-ether; Perfluor-di-n-butylether; Trichlor-trifluorethan.

Wenn langkettige 1-Iodperfluoralkane (ab 1-Iodperfluordecan) eingesetzt werden, kann das Reaktionsgemisch feste Bestandteile enthalten. Hier gibt man am besten vor der Phasentrennung mindestens ein Lösungsmittel zu, welches die obengenannten Eigenschaften besitzt, beispielsweise 1,1,2-Trichlortrifluorethan.

Die nach dem Abkühlen gebildeten zwei flüssigen Phasen werden nach üblichen Methoden, beispielweise durch Dekantieren oder vermittels eines Scheidetrichters, voneinander getrennt.

Die leichtere Phase wird mit 0,3 bis 6% ihres Gewichtes Wasser oder einer entsprechenden Menge einer wasserhaltigen Flüssigkeit intensiv vermischt, beispielsweise durch Schütteln oder Rühren. Als wasserhaltige Flüssigkeit wird vorzugsweise eine Mischung von Schwefelsäure und Wasser verwendet. Wurde zur Reaktionsmischung und/oder zur abgetrennten leichteren Phase, wie oben beschrieben, ein Lösungsmittel gegeben, so ist dessen Gewicht vom Gewicht der leichteren Phase bei Feststellung der Wasser-Zugabe-Menge abzuziehen.

In einer bevorzugten Ausführungsform wird der Gehalt der abgetrennten leichteren Phase an Perfluorcarbonsäurefluorid analytisch ermittelt, beispielsweise durch Mischen einer Probe mit Wasser und Bestimmen der gebildeten Fluoridionen mit einer ionen-sensitiven Elektrode oder durch das $^{19}$F-Kernresonanzspektrum. Aufgrund des ermittelten Wertes wird die stöchiometrisch notwendige Wassermenge zur Zersetzung des Säurefluorids festgestellt und 105 bis 130% dieser Wassermenge der leichteren Phase, wie oben beschrieben, zugemischt.

Vorteilhaft ist ferner, die Mischung der leichteren Phase mit Wasser oder einer wasserhaltigen Flüssigkeit auf 40 bis 100°C zu erwärmen, insbesondere, wenn Perfluorcarbonsäuren mit 8 und mehr C-Atomen hergestellt werden. Durch die Erwärmung wird die Zersetzung des Säurefluorids beschleunigt und vervollständigt. Das gegebenenfalls vorhandene organische, fluorierte Lösungsmittel kann vor oder nach der Hydrolyse durch Verdampfen entfernt werden. Die reine Perfluorcarbonsäure wird schließlich durch fraktionierende Destillation des Umsetzungsproduktes der leichteren Phase mit Wasser gewonnen, wobei je nach Kettenlänge der gebildeten Perfluorcarbonsäure bei 0,1 bis 100 kPa Druck destilliert wird.

Wegen des bei der Umsetzung der leichteren Phase mit Wasser entstehenden feuchten Fluorwasserstoffs müssen Hydrolyseapparatur und Destillationskolonne aus korrosionsbeständigem Material bestehen oder damit ausgekleidet sein. Solche Materialien sind beispielsweise fluorhaltige Polymere oder Copolymere, zum Beispiel Polytetrafluorethylen, Polyvinylidenfluorid oder Tetrafluorethylen-Ethylen-Copolymere sowie Edelmetalle, wie Tantal oder Platin.

Es wurde gefunden, daß die aus der Reaktion mit 1-lodperfluoralkan erhaltene, als schwerere Phase abgetrennte rauchende Schwefelsäure sich nicht einfach dadurch wiederverwenden läßt, daß man das verbrauchte Schwefeltrioxid ersetzt und filtriert, wie in DE-PS 1 211 619 beschrieben. Die gelösten anorganischen Reaktionsprodukte erreichen bald ein unvertretbar hohes Maß. Eine solche rauchende Schwefelsäure wirkt bereits nach einmaligem Gebrauch metallkorrodierend, das während der Reaktion gebildete lod wird so nur sehr unvollkommen erhalten. Es ist aber wünschenswert, das in gelöster Form vorliegende lod möglichst quantitativ als elementares lod zurückzugewinnen, da es als solches zur Herstellung der lodperfluoralkan-Ausgangsverbindungen benötigt wird.

Beim Erhitzen der gebrauchten rauchenden Schwefelsäure im Vakuum werden als flüchtige Anteile Schwefeltrioxid und Fluorsulfonsäure erhalten, jedoch nur wenig lod. Läßt man vorgelegtes Wasser oder verdünnte Schwefelsäure mit der rauchenden Schwefelsäure unter Kühlung reagieren, wobei die Temperatur unter 50°C bleibt, so ist die lodfällung unvollständig, selbst wenn man die Mischung anschließend mit starken Oxidationsmitteln wie Chlor oder Wasserstoffperoxid behandelt.

Überraschend wurde jedoch gefunden, daß sich das gelöste lod in sehr guter Ausbeute als elementares lod abscheidet, wenn man die als schwerere Phase aus dem Reaktionsgemisch abgetrennte rauchende Schwefelsäure mit 0,7 bis 1,5 Mol Wasser oder einer entsprechenden Menge einer wasserhaltigen Flüssigkeit, beispielsweise wasserhaltiger Schwefelsäure, je Mol eingesetztes Schwefeltrioxid, gegebenenfalls unter zusätzlicher Erwärmung, vermischt.

Werden weniger als 0,7 Mol Wasser je Mol Schwefeltrioxid verwendet, verschlechtert sich die Ausbeute des abgeschiedenen elementaren lods. Bei über 1,5 Mol Wasser je Mol Schwefeltrioxid wird die rauchende Schwefelsäure unnötig verdünnt. Ihr Wiedereinsatz wird unwirtschaftlicher, da größere Mengen Schwefeltrioxid gebraucht werden, um eine für die Reaktion mit 1-lodperfluoralkan geeignete rauchende Schwefelsäure zu erhalten. Vorzugsweise werden 0,9 bis 1,2 Mol Wasser oder wasserhaltige Flüssigkeit je Mol Schwefeltrioxid verwendet.

Wird Wasser oder eine stark wasserhaltige Flüssigkeit, beispielsweise verdünnte Schwefelsäure, verwendet, so wird diese(s) zweckmäßig vorgelegt und die abgetrennte schwerere Phase unter Bewegung der Mischung durch Rühren oder Schütteln zugegeben. Wenn höher konzentrierte, beispielsweise 70 bis 80gew.-%ige Schwefelsäure als wasserhaltige Flüssigkeit verwendet wird, kann diese langsam unter Bewegen der Mischung zur abgetrennten schwereren Phase gegeben werden.

Bei der Wasser-Zugabe erwärmt sich die Mischung. Die Temperatur der Mischung soll mindestens 50°C erreichen, um eine gute Ausbeute an abgeschiedenem, elementarem lod zu erhalten. Vorteilhaft werden Temperaturen der Reaktionsmischung von 70 bis 100°C, gegebenenfalls durch zusätzliche Erwärmung oder Kühlung eingestellt. Über 100°C tritt in zunehmendem Maße eine unerwünschte Verdampfung des ausgeschiedenen lods ein.

Bei Zugabe des Wassers oder der wasserhaltigen Flüssigkeit entweichen aus der Mischung gasförmiges Schwefeldioxid und ein großer Teil des Fluors, das in der, wie oben beschrieben, abgetrennten schwereren flüssigen Phase enthalten war, als Fluorwasserstoff. Die entweichenden Gase werden zweckmäßig durch Einleiten in Kalkmilch aus dem Abgas entfernt.

Nach Beendigung der Reaktion mit Wasser, die je nach Wassermenge und eingestellter Temperatur etwa 10 Minuten bis 1½ Stunden dauert, wird die Mischung auf 0 bis +30°C abgekühlt. Über +30°C ist die Ausscheidung von lod unvollständiger und das lod schwerer. filtrierbar. Die untere Temperaturgrenze der Abkühlung ist dadurch gegeben, daß ein Auskristallisieren der Schwefelsäure oder ihrer Hydrate weitgehend vermieden werden soll.

Aus der abgekühlten Mischung scheidet sich nahezu quantitativ elementares, festes lod aus. Es wird nach üblichen Methoden, beispielsweise durch Filtrieren oder Zentrifugieren von der flüssigen Phase abgeschieden, zweckmäßig zunächst mit wasserhaltiger Schwefelsäure im Konzentrationsbereich 80 bis 30 Gew.-% $H_2SO_4$, dann mit reinem Wasser gewaschen. Das so erhaltene lod kann anschließend nach bekannten Verfahren getrocknet und beispielsweise durch Destillation oder Sublimation gereinigt werden.

Die nach Abtrennung des lods verbleibende flüssige Phase wird vorteilhaft mit Schwefeltrioxid oder mit rauchender Schwefelsäure, die einen hohen Schwefeltrioxidgehalt, beispielsweise 50 bis 70 Gew.-%, aufweist, versetzt, in einer Menge, daß die gesamte Mischung einen Gehalt an Schwefeltrioxid von 15 bis 45 Gew.-% aufweist. Diese Mischung wird zur Umsetzung weiterer 1-lodperfluoralkans verwendet.

Das in den vorangegangenen Seiten beschriebene neue Verfahren ist besonders vorteilhaft durchführbar bei der Umsetzung von 1-lodperfluor-n-octan oder von Gemischen verschiedener 1-lodperfluoralkyl-Verbindungen, die einen überwiegenden Anteil an Verbindungen mit 6 bis 14 C-Atomen und mindestens 25 Gew.-% 1-Iodperfluor-n-octan enthalten mit rauchender Schwefelsäure.

Das erfindungsgemäße Verfahren ermöglicht es, insbesondere geradkettige Perfluorcarbonsäuren und speziell die technisch wichtige Perfluoroctansäure sowie elementares Iod in hohen Ausbeuten und hoher Reinheit zu gewinnen. Es gestattet ferner, die Schwefelsäure zurückzugewinnen und im Kreis zu führen, wobei die Umsetzung der rauchenden Schwefelsäure mit dem 1-Iodperfluoralkan ohne stärkere Korrosion in Stahlgefäßen erfolgen kann.

Die folgenden Beispiele sollen das erfindungsgemäße Verfahren näher erläutern.

## Beispiel 1

### Herstellung von Perfluoroctansäure

Ein 2-l-Rührautoklav aus V2A-Stahl wird mit $N_2$ gespült und dann mit 900 g 1-Iodperfluor-n-octan ($n-C_8F_{17}I$, 1,648 Mol) und 1700 g rauchender Schwefelsäure mit 35 Gew.-% (= 7,44 Mol) freiem Schwefeltrioxid (876 ml 35%iges Oleum) beschickt und verschlossen. Das Molverhältnis Schwefeltrioxid zu 1-Iodperfluoralkan beträgt 4,5 : 1. Man heizt innerhalb von 90 Minuten auf 155°C auf, rührt 4 Stunden bei dieser Temperatur und kühlt anschließend auf Raumtemperatur ab. Man entleert den Autoklaven in einen Scheidetrichter und trennt nach einstündigem Stehen die entstandenen Phasen. Als untere, schwerere Phase erhält man 1900 g dunkelbraun gefärbte, gebrauchte rauchende Schwefelsäure. Als obere, leichtere blaßrosa gefärbte Phase fallen 670 g einer Mischung von Fluorverbindungen an, welche gemäß $^{19}$F-Kernresonanzspektrum aus je 48 Mol-% $n-C_7F_{15}COF$ und $n-C_7F_{15}COOH$ sowie aus nicht umgesetztem $n-C_8F_{17}I$ (ca. 3 Mol-%) besteht.

Zur leichteren Phase werden in eine mit Polyvinylidenfluorid-Folie ausgekleidete und mit einem Polytetrafluorethylen-Rührer versehene Apparatur bei 60°C 15 g Wasser (= 2,2% des Gewichtes der leichteren Phase) gegeben und kräftig gerührt. Das entweichende fluorwasserstoff-haltige Gas wird in eine Calciumhydroxid-Suspension eingeleitet. Nach 30 Minuten ist die Gas-Entwicklung beendet. Das so erhaltene Rohprodukt wird bei 5,3 kPa fraktioniert destilliert. Man erhält 20 g eines Vorlaufs bis Siedepunkt 113°C, der im wesentlichen aus 1-Iodperfluor-n-octan besteht, und 648 g farblose, reine Perfluoroctansäure(1) (Siedepunkt 113°C/5,3 kPa, Schmelzpunkt 54 bis 55°C). Die Ausbeute beträgt 98%, bezogen auf umgesetztes $n-C_8F_{17}I$.

In einer Rührapparatur läßt man zur abgetrennten schwereren Phase unter kräftigem Rühren während 30 Minuten 770 g 80gew.-%ige Schwefelsäure zutropfen. Diese enthält 154 g (= 8,55 Mol) Wasser. Je Mol ursprünglich eingesetztes Schwefeltrioxid werden 1,15 Mol Wasser verwendet. Die Temperatur wird auf 85°C eingestellt. Entweichende Gase (Schwefeldioxid und Fluorwasserstoff) werden mit Kalkmilch neutralisiert. Die Mischung wird auf 20°C abgekühlt und das abgeschiedene feinkristalline Iod wird mit Hilfe einer Glasfilternutsche von der Schwefelsäure getrennt und nacheinander mit 200 ml 80gew.-%iger $H_2SO_4$, dann mit 200 ml 50gew.-%iger $H_2SO_4$ und schließlich mit 500 ml $H_2O$ gewaschen. Man erhält 209 g feuchtes Iod (Wassergehalt 4 Gew.-%), entsprechend einer Wiedergewinnung von 99%, bezogen auf das umgesetzte $n-C_8F_{17}I$.

## Beispiele 2 bis 9

In Tabelle 1 sind die Ergebnisse kleinerer Ansätze zusammengefaßt, die mit rauchender Schwefelsäure, die 35 Gew.-% Schwefeltrioxid enthält, unter Variation von $SO_3$ : $n-C_8F_{17}I$-Molverhältnis und Reaktionszeit erhalten werden. Die Aufarbeitung erfolgt analog Beispiel 1.

Man erkennt aus den Beispielen 8 und 9, daß ein hohes Schwefeltrixod : $n-C_8F_{17}I$-Molverhältnis zwar zu hohen Umsetzungsgraden führt, daß sich jedoch die Carbonsäure-Ausbeute durch Bildung von Nebenprodukten ($C_8F_{17}H$, $C_6F_{13}CF=CF_2$, $C_6F_{13}CO_2H$) deutlich vermindert.

0 052 285

Tabelle 1

Umsetzungen von n-C$_8$F$_{17}$I mit rauchender Schwefelsäure mit 35 Gew.-% Schwefeltrioxid-Gehalt bei 155°C*)

| Beispiel Nr. | Einsatzmengen | | Molverhältnis | Reaktions-dauer | Umsatz | n-C$_7$F$_{15}$CO$_2$H-Ausbeute |
|---|---|---|---|---|---|---|
| | n-C$_8$F$_{17}$I | Schwefel-säure + SO$_3$ | SO$_3$/n-C$_8$F$_{17}$I | | | |
| | (g) | (g) | | (h) | (%) | (%) |
| 2 | 100 | 95 | 2,27 | 4 | 47 | 38 |
| 3 | 100 | 142 | 3,39 | 4 | 72 | 63 |
| 4 | 100 | 142 | 3,39 | 6 | 78 | 70 |
| 5 | 100 | 142 | 3,39 | 8 | 83 | 75 |
| 6 | 100 | 190 | 4,54 | 4 | 91 | 82 |
| 7 | 100 | 190 | 4,54 | 6 | 96 | 87 |
| 8 | 100 | 390 | 9,32 | 4 | 96 | 78 |
| 9 | 100 | 600 | 14,33 | 4 | 98 | 72 |

*) Durchgeführt in V4A-Schüttelautoklaven. Aufheiz- und Abkühlzeit jeweils 1 Stunde. Die Perfluoroctansäure-Ausbeuten (destillierte Säure) beziehen sich auf eingesetztes n-C$_8$F$_{17}$I.

Beispiel 10

Wiederverwendung der bei der Iodgewinnung anfallenden konzentrierten Schwefelsäure

In 1005 g der gemäß Beispiel 1 nach der Abtrennung von Iod erhaltenen Schwefelsäure werden 700 g Schwefeltrioxid gelöst. Die auf diese Weise gebildeten 1705 g rauchende Schwefelsäure mit etwa 35 Gew.-% Schwefeltrioxid werden wie in Beispiel 1 mit 900 g n-C$_8$F$_{17}$I umgesetzt. Die auf n-C$_8$F$_{17}$I-Umsatz bezogenen Ausbeuten sind 645,6 g n-C$_7$F$_{15}$CO$_2$H (97,5%) und 210,2 g feuchtes Iod (H$_2$O-Gehalt 4,7%, Ausbeute 98,7%).

Weitere 630 g der gemäß Beispiel 1 erhaltenen End-Schwefelsäure werden mit 140 g Wasser versetzt. Diese 770 g 80gew.-%ige Schwefelsäure werden gemäß Beispiel 1 wiederverwendet.

Beispiel 11

Perfluorhexansäure

111,5 g (= 0,25 Mol) 1-Iodperfluor-n-hexan (n-C$_6$F$_{13}$I) und 260 g rauchende Schwefelsäure mit 35 Gew.-% Schwefeltrioxid (= 1,14 Mol) werden in einem Schüttelautoklaven während 90 Minuten auf 155°C aufgeheizt und 3,5 Stunden bei dieser Temperatur geschüttelt. Das Molverhältnis Schwefeltrioxid zu 1-Iodperfluoralkan beträgt 4,6 : 1. Nach Abkühlen des Autoklaven werden die Phasen getrennt. Es werden erhalten: 70,3 g leichtere Phase und 283,9 g schwerere Phase. Durch dreimaliges Ausschütteln der schwereren Phase mit je 40 ml 1,1,2-Trichlor-trifluorethan und Abdampfen des Extraktionsmittels erhält man 5,5 g flüssigen Rückstand, der mit der leichteren Phase vereinigt wird. Diese enthält nach $^{19}$F-Kernresonanzspektrum: 5 Mol-% n-C$_6$F$_{13}$I, 43 Mol-% n-C$_5$F$_{11}$COF, 47 Mol-% n-C$_5$F$_{11}$CO$_2$H, der Rest sind unbekannte Verbindungen (wahrscheinlich n-C$_6$F$_{13}$OSO$_2$F). Die leichtere Phase wird mit 2,8 g Wasser (= 4% des Gewichtes der leichteren Phase) Wasser bei 20°C intensiv vermischt. Nach Beendigung der Gasentwicklung ergibt die fraktionierte Destillation bei Normaldruck 7,5 g Vorlauf und 60,6 g reine Perfluorhexansäure(1) (Siedepunkt 152 bis 153°C, Ausbeute 81%, bezogen auf umgesetztes n-C$_6$F$_{13}$I).

Die abgetrennte und wie oben beschrieben extrahierte schwerere Phase wird unter kräftigem Rühren während 20 Minuten mit 35 g einer 50gew.-%igen Schwefelsäure versetzt. Diese enthält 17,5 g

6

(= 0,97 Mol) Wasser. Je Mol ursprünglich eingesetztes Schwefeltrioxid werden 0,85 Mol Wasser verwendet. Durch Erwärmen wird die Temperatur auf 95° C eingestellt. Entweichende Gase werden mit Kalkmilch neutralisiert.

Die Mischung wird auf 12° C abgekühlt, das abgeschiedene Iod abfiltriert und wie im Beispiel 1 beschrieben gewaschen. Es werden 30 g feuchtes Iod (Wassergehalt 4 Gew.-%) erhalten, entsprechend einer Wiedergewinnung von 95,5%, bezogen auf das umgesetzte n-$C_6F_{13}$I.

Beispiel 12

Perfluordecansäure

129,2 g 1-Iodperfluor-n-decan (n-$C_{10}F_{21}$I, 0,2 Mol) und 207,7 g rauchende Schwefelsäure mit 35 Gew.-% Schwefeltrioxid (= 0,91 Mol) werden in einem Stahlautoklaven 8 Stunden bei 155° C geschüttelt. Das Molverhältnis Schwefeltrioxid zu 1-Iodperfluoralkan beträgt 1 : 4,6. Nach Abkühlen auf Raumtemperatur setzt man 50 ml (= 78,8 g 1,1,2-Trichlor-trifluorethan zu, schüttelt nochmals kräftig und trennt die Phasen. Man erhält 186 g leichtere Phase. Diese enthält nach $^{19}$F-Kernresonanz-Spektrum neben dem Lösungsmittel 62 Mol-% n-$C_9F_{19}$COF, 21 Mol-% n-$C_9F_{19}CO_2$H und 17 Mol-% n-$C_{10}F_{21}$I. Zur leichteren Phase werden 2,5 g $H_2O$ (= 2,3% des Gewichtes dieser Phase ohne Lösungsmittel) gegeben und die Mischung wird 20 Minuten unter Rückfluß auf ca. 55° C erhitzt. Nach Abdestillieren des Lösungsmittels erhält man durch fraktionierende Destillation 25 g Vorlauf (Siedepunkt 103 bis 149° C/9,3 kPa) und 72,9 g Hauptfraktion (Siedepunkt 149 bis 153° C/9,3 kPa). Unter Vernachlässigung der im Vorlauf enthaltenen Säuremenge beträgt die n-Perfluordecansäure-Ausbeute 85,1%, bezogen auf den n-$C_{10}F_{21}$I-Umsatz von 83%.

Die abgetrennte, schwerere Phase wird unter kräftigem Rühren zu 24 g (= 1,33 Mol) Wasser gegeben. Je Mol ursprünglich eingesetztes Schwefeltrioxid werden 1,47 Mol Wasser verwendet. Es wird eine Temperatur von 73° C eingestellt. Entweichende Gase werden mit Kalkmilch neutralisiert.

Die Mischung wird auf 8° C abgekühlt, das abgeschiedene Iod abfiltriert und, wie in Beispiel 1 beschrieben, gewaschen. Es werden 21,2 g feuchtes Iod (Wassergehalt 4 Gew.-%) erhalten, entsprechend einer Wiedergewinnung von 96,5%, bezogen auf das umgesetzte n-$C_{10}F_{19}$I.

Beispiel 13

Herstellung eines Perfluorcarbonsäure-Gemisches

115,3 g (0,22 Mol) eines 1-Iodperfluoralkan-Gemisches, welches aus 43,5% n-$C_6F_{13}$I, 33,0% n-$C_8F_{17}$I, 16,5% n-$C_{10}F_{21}$I und 7,0% n-$C_{12}F_{25}$I besteht, werden mit 228 g rauchender Schwefelsäure mit 35 Gew.-% Schwefeltrioxid (= 1 Mol) 6 Stunden bei 155° C in einem Schüttelautoklaven umgesetzt. Das Molverhältnis Schwefeltrioxid zu 1-Iodperfluoralkan beträgt 4,5 : 1.

Nach Abkühlen und Phasentrennung erhält man 93,2 g einer leichteren Phase mit der Zusammensetzung: 26 Mol-% Perfluoralkansäurefluoride, 63,0 Mol-% Perfluoralkansäuren(1), 6,5 Mol-% nicht-umgesetzte 1-Iodperfluoralkane und 4,5 Mol-% Verbindungen der Formel $R_FOSO_2$F. Vermischung der leichteren Phase mit 3 g (= 3,2% des Gewichtes dieser Phase) Wasser bei einer Anfangstemperatur von 20° C und einer Endtemperatur von 80° C und anschließender Destillation liefert 7 g Vorlauf (Siedepunkt 80 bis 149° C) und 66,8 g Hauptfraktion, welche zwischen 113° C/13,3 kPa und 145° C/6,7 kPa übergeht. Die auf 93,5% Umsatz bezogene Ausbeute an Perfluoralkancarbonsäure-Gemisch $C_6$ bis $C_{12}$ beträgt 82,8%.

Die analog Beispiel 1 durchgeführte Iodrückgewinnung aus der schwereren Phase ergibt eine Ausbeute von 97,2% Iod, bezogen auf umgesetztes Iodperfluoralkan.

Patentansprüche

1. Verfahren zur Reindarstellung gesättigter Perfluoralkancarbonsäuren(1) mit 6 bis 14 C-Atomen aus den entsprechenden 1-Iodperfluoralkanen unter weitgehender Rückgewinnung von Iod durch Umsetzung der 1-Iodperfluoralkane mit rauchender Schwefelsäure, die 15 bis 45 Gew.-%, bezogen auf die Lösung, Schwefeltrioxid gelöst enthält, bei Temperaturen von 100 bis 180° C, gegebenenfalls unter Druck, wobei das Molverhältnis von Schwefeltrioxid zu 1-Iodperfluoralkan 2 : 1 bis 15 : 1 beträgt und nach beendeter Reaktion die entstandenen zwei flüssigen Phasen voneinander getrennt werden, dadurch gekennzeichnet, daß man die leichtere flüssige Phase mit 0,3 bis 6% ihres Gewichtes Wasser oder einer entsprechenden Menge einer wasserhaltigen Flüssigkeit, gegebenenfalls bei erhöhter Temperatur, vermischt und anschließend diese Mischung bei 0,1 bis 100 kPa Druck destilliert, ferner die nach der Reaktion mit rauchender Schwefelsäure abgetrennte schwerere flüssige Phase mit 0,7 bis 1,5 Mol Wasser oder einer entsprechenden Menge einer wasserhaltigen Schwefelsäure je Mol

7

eingesetztes Schwefeltrioxid, gegebenenfalls unter zusätzlicher Erwärmung, vermischt, danach auf 0 bis +30° C abkühlt, das ausgeschiedene feste Iod abfiltriert, mit wasserhaltiger Schwefelsäure sowie mit Wasser wäscht, gegebenenfalls trocknet und nach bekannten Methoden reinigt.

2. Verfahren nach Anspruch 1, dadurch gekennzeichnet, daß man die nach Abtrennung des Iods anfallende konzentrierte Schwefelsäure mit so viel Schwefeltrioxid versetzt, daß eine Lösung mit 15 bis 45 Gew.-% Schwefeltrioxid entsteht und diese Lösung zur Umsetzung mit weiterem 1-Iodperfluoralkan verwendet wird.

3. Verfahren nach einem der Ansprüche 1 oder 2, dadurch gekennzeichnet, daß man die abgetrennte leichtere flüssige Phase nach dem Wasserzusatz auf 40 bis 100° C erwärmt.

4. Verfahren nach einem der Ansprüche 1 bis 3, dadurch gekennzeichnet, daß man die Temperatur der abgetrennten und mit Wasser versetzten schwereren flüssigen Phase auf 70 bis 100° C einstellt.

5. Verfahren nach einem der Ansprüche 1 bis 4, dadurch gekennzeichnet, daß man 1-Iodperfluor-n-octan oder Gemische von 1-Iodperfluoralkyl-Verbindungen, die einen Anteil an 1-Iodperfluor-n-octan von mindestens 25 Gew.-%, bezogen auf die Mischung, enthalten, mit rauchender Schwefelsäure umsetzt.

6. Verfahren nach einem der Ansprüche 1 bis 5, dadurch gekennzeichnet, daß bei der Umsetzung von 1-Iodperfluoralkan mit rauchender Schwefelsäure das Molverhältnis von Schwefeltrioxid zum 1-Iodperfluoralkan 3 : 1 bis 6 : 1 beträgt.

## Claims

1. A process for the preparation of pure saturated perfluoro-1-carboxylic acids, having 6 to 14 C atoms from the corresponding 1-iodoperfluoroalkanes, with substantial recovery of iodine, by reacting the 1-iodoperfluoroalkanes with fuming sulfuric acid containing 15 to 45% by weight, relative to the solution, of dissolved sulfur trioxide, at temperatures of 100 to 180° C, if appropriate under pressure, the molar ratio of sulfur trioxide to 1-iodoperfluoroalkane being 2 : 1 to 15 : 1 and the resulting two liquid phases being separated from one another when the reaction is complete, characterised by mixing, if necessary at elevated temperatures, the lighter liquid phase with 0.3 to 6% of its weight of water or with a corresponding amount of an aqueous liquid, and then distilling this mixture at a pressure of 0.1 to 100 kPa, and also mixing the heavier liquid phase which has been separated off after the reaction with fuming sulfuric acid, with 0.7 to 1.5 moles of water, or with a corresponding amount of an aqueous sulfuric acid, per mole of sulfur trioxide employed, if necessary warming it additionally, then cooling to 0 to 30° C, filtering off the solid iodine which has been precipitated, washing it with aqueous sulfuric acid and with water and, optionally drying it and purifying it by known methods.

2. A process as claimed in claim 1, characterised by adding sufficient sulfur trioxide to the concentrated sulfuric acid produced after the removal of iodine to give a solution containing 15 to 45% by weight of sulfur trioxide, and using this solution for reacting with further 1-iodoperfluoroalkane.

3. A process as claimed in either of claims 1 or 2, characterised by warming the lighter liquid phase which has been separated off after water has been added to it to 40 to 100° C.

4. A process as claimed in either of claims 1 to 3, characterised by adjusting the temperature of the heavier liquid phase which has been separated off and to which water has been added to 70 to 100° C.

5. A process as claimed in either of claims 1 to 4, characterised by reacting with fuming sulfuric acid 1-iodoperfluoro-n-octane or mixtures of 1-iodoperfluoroalkyl compounds containing a proportion, relative to the mixture, of at least 25% by weight of 1-iodoperfluoro-n-octane.

6. A process as claimed in either of claims 1 to 5, characterised in that the molar ratio of sulfur trioxide to 1-iodoperfluoroalkane in the reaction of 1-iodoperfluoroalkane with fuming sulfuric acid is 3 : 1 to 6 : 1.

## Revendications

1. Procédé de préparation à l'état pur d'acides perfluoroalcane-1-carboxyliques saturés comportant 6 à 14 atomes de carbone à partir des iodo-1 perfluoroalcanes correspondants, avec récupération dans une large mesure de l'iode, par réaction des iodo-1 perfluoroalcanes avec de l'acide sulfurique fumant qui contient 15 à 45% en poids, par rapport à la solution, de trioxyde de soufre dissous, à des températures de 100 à 180° C, éventuellement sous pression, procédé selon lequel le rapport molaire du trioxyde de soufre au iodo-1 perfluoroalcane se situe entre 2 : 1 et 15 : 1 et, après achèvement de la réaction, on sépare l'une de l'autre les deux phases liquides résultantes, procédé caractérisé en ce qu'on mélange à la phase liquide légère 0,3 à 6% de son poids d'eau ou une quantité correspondante d'un liquide aqueux, éventuellement à température élevée, et l'on distille ensuite ce mélange sous une pression de 0,1 à 100 kPa; on mélange en outre, éventuellement sous chauffage supplémentaire, à la phase liquide lourde séparée après la réaction avec l'acide sulfurique fumant 0,7 à 1,5 mole d'eau ou une quantité correspondante d'un acide sulfurique aqueux, par mole de trioxyde de soufre mis en oeuvre; puis l'on refroidit jusqu'à 0 à +30° C, on sépare par filtration l'iode solide précipité, on le lave

**0 052 285**

avec de l'acide sulfurique aqueux et avec de l'eau, on le sèche éventuellement et on le purifie d'après des méthodes connues.

2. Procédé selon la revendication 1, caractérisé en ce qu'on ajoute à l'acide sulfurique concentré, obtenu après séparation de l'iode, une quantité de trioxyde de soufre permettant d'obtenir une solution contenant 15 à 45% en poids de trioxyde de soufre et l'on utilise cette solution pour la faire réagir avec un supplément d'iodo-1 perfluoroalcane.

3. Procédé selon l'une des revendications 1 ou 2, caractérisé en ce qu'après l'addition d'eau, on chauffe jusqu'à 40 à 100°C la phase liquide légère séparée.

4. Procédé selon l'une quelconque des revendications 1 à 3, caractérisé en ce qu'on règle entre 70 et 100°C la température de la phse liquide lourde séparée et additionnée d'eau.

5. Procédé selon l'une quelconque des revendications 1 à 4, caractérisé en ce qu'on fait réagir avec l'acide sulfurique fumant de l'iodo-1 perfluoro-octane normal ou des mélanges de composés iodo-1 perfluoro alkylés qui contiennent une proportion d'au moins 25% en poids, par rapport au mélange, de l'iodo-1 perfluoro-octane normal.

6. Procédé selon l'une quelconque des revendications 1 à 5, caractérisé en ce que, lors de la réaction de l'iodo-1 perfluoroalcane avec l'acide sulfurique fumant, le rapport molaire du trioxyde de soufre à l'iodo-1 perfluoroalcane se situe entre 3 : 1 et 6 : 1.

9